# EUROPEAN PATENT SPECIFICATION

(11) **EP 3 053 575 B1**
(45) Date of publication and mention of the grant of the patent: **02.09.2020**
(21) Application number: 14851001.9
(22) Date of filing: 03.10.2014
(51) Int. Cl.: A61K 31/558, A61K 9/00, A61P 13/12

(54) **AGENT FOR PREVENTING OR AMELIORATING RENAL DYSFUNCTION**
MITTEL ZUR VERHINDERUNG ODER LINDERUNG VON NIERENFUNKTIONSSTÖRUNGEN
AGENT SERVANT À PRÉVENIR OU AMÉLIORER UN DYSFONCTIONNEMENT RÉNAL

(30) Priority: 04.10.2013 JP 2013209539
(43) Date of publication of application: 10.08.2016
(73) Proprietor: Tohoku University, Sendai-shi, Miyagi 980-8577 (JP)
(72) Inventor: ABE, Takaaki, Sendai-shi Miyagi 980-8577 (JP)
(74) Representative: Mewburn Ellis LLP
(86) International application number: PCT/JP2014/005049
(87) International publication number: WO 2015/049876

(56) References cited:
- WO-A1-2012/144649
- JP-A- H0 770 054
- JP-A- H04 330 015
- JP-A- 2005 504 836
- JP-A- 2010 538 016
- US-A1- 2010 215 779
- E. MISHIMA ET AL: "Alteration of the Intestinal Environment by Lubiprostone Is Associated with Amelioration of Adenine-Induced CKD", JOURNAL OF THE AMERICAN SOCIETY OF NEPHROLOGY., vol. 26, no. 8, 18 December 2014 (2014-12-18), pages 1787-1794, XP055365214, US ISSN: 1046-6673, DOI: 10.1681/ASN.2014060530

## Description

### Technical Field

The present invention relates to lubiprostone or its pharmacologically acceptable salt for use in preventing or ameliorating renal dysfunction.

### Background Art

The kidney is a very important organ that maintains the body's homeostasis by regulating the concentration of body fluid components through excretion/resorption and also functions as an endocrine organ producing/secreting physiologically active substances, such as active vitamin D, erythropoietin, and renin. Compromise in the function of the kidney playing such important roles causes various pathological conditions and produces a wide variety of renal diseases. These renal diseases include nephropathies intimately associated with diabetes, obesity, and lipid metabolism abnormality; particularly, diabetic nephropathy due to diabetes finds difficulty in arresting its advance after the diagnosis of the neuropathy even when the diabetes is strictly controlled, results in renal failure in many cases, and is not small in the number of patients.

There is no particularly effective drug therapy for renal failure in which renal function is highly compromised; dialysis accompanied by distress is performed on patients with renal failure; and the number of dialysis patients is reported to have exceeded 300,000 in 2012. Dialysis generally requires the visit of patients to dialysis facilities several times a week and the constraint of them for several hours and involves large expense, and thus is well known to psychologically and physically give a severe burden to the patients. In addition, the removal of metabolic waste products and water by dialysis can never be said to be sufficient; thus, there is need for the development of an agent for preventing the decline in renal function (an agent for improving renal function) in view of preventing renal failure in advance.

Many currently known agents for improving renal function have strong side effects and have limited therapeutic effects. For example, although some dietary fibers, such as gum arabic, and oligosaccharides are known to have the promotion effect on nitrogen excretion (Non-patent Documents 1 and 2), they are difficult to ingest for a long period of time in view of taste, and not suitable for continued treatment since nausea, bloating sensation, and diarrhea due to the ingestion of them in large amounts are reported. A urea nitrogen metabolism-improving agent is disclosed which contains, as an active ingredient, at least one selected from the group consisting of flavonoids, saponins, and their glucosides contained in leguminous plants (Patent Document 1). However, such an improvement agent is difficult to produce and has many unclear points in its action mechanism, and ambiguous points remain in the confirmation of its effect. A renal function-improving agent is also disclosed which contains a fermentation metabolite of bifidobacteria as an active ingredient (Patent Document 2). In addition, a renal disease-ameliorating agent/food ("Nisshoku selfer (R)" from Nihon Shokuhin Kako Co., Ltd.) is disclosed which contains a dietary fiber obtained from the hull of a food seed as an active ingredient (Patent Document 3). However, for this renal disease-ameliorating agent, the result is only shown that the survival time of renal failure rats was 2.7 days in a control group while being extended to 3.4 days by the administration of the test substance, and its effect as a renal disease-ameliorating agent is not quite clarified.

A phosphate binder is also disclosed which contains chitosan as an active ingredient (Patent Document 4). However, although it is useful for the treatment of hyperphosphatemia secondarily caused as a result of renal disease but has no effect for ameliorating renal disease itself. In addition, disclosed are, for example, a low-potassium and low-phosphorus soybean protein for renal disease patients (Patent Document 5), a dietary fiber having a reduced electrolyte content (Patent Document 6), and a dietary fiber composition having a reduced phosphorus/potassium content (Patent Document 7); however, they also only ameliorate some of secondarily occurring symptoms (e.g., hyperphosphatemia and hyperkalemia), and are not observed to have clinical effects.

Thus many techniques and agents relating to an agent for ameliorating symptoms of renal disease are disclosed; however, no effective agent is present which has the suppressive action on the accumulation of waste products in the body of renal disease patients and promoting their excretion and thus there is a need for the development of a more effective and safe renal function-improving agent.

Meanwhile, lubiprostone, i.e., (-)-7-[(2R,4aR,5R,7aR)-2-(1,1-difluoropentyl)-2-hydroxy-6-oxooctahydrocyclopenta[b]pyran-5-yl]heptanoic acid, is a prostaglandin compound, and is known to be typically present in the form of the following tautomer (Patent Document 8).

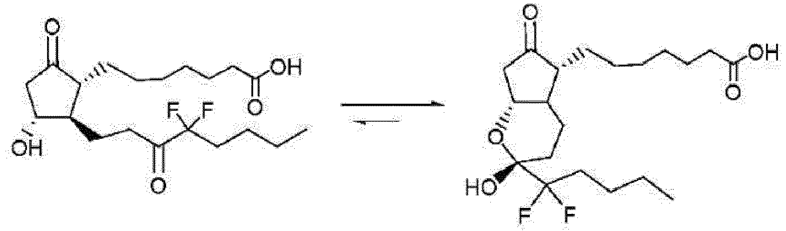

Lubiprostone has the amelioration effect on constipation, in which the water content of feces is lowered, by activating CIC-2 chloride channel present in the epithelium of the small intestine and promoting the excretion of water (Patent Document 8 and Non-patent Documents 3 and 4). Clinical studies of lubiprostone

(trade name: Amitiza (R)) capsules were started in US by Sucampo Pharmaceuticals Inc. as a US subsidiary of Sucampo Pharma Inc., and the lubiprostone capsules are now approved as a therapeutic agent for chronic idiopathic constipation in Japan in addition to US and Switzerland. However, it was unclear whether lubiprostone had the prevention or amelioration effect on renal dysfunction.

US 2010/0215779 describes the use of lubiprostone to reduce fluid and/or salt retention by reducing sodium absorption in the intestine and/or increasing anion secretion.

WO 2012/144649 describes the use of lubiprostone in modulating cytokine activity in a mammal.

### Prior Art Documents

### Patent Documents

Patent Document 1: Japanese Patent Publication No. 08-32632
Patent Document 2: Japanese unexamined Patent Application Publication No. 01-163128
Patent Document 3: Japanese unexamined Patent Application Publication No. 02-101016
Patent Document 4: Japanese unexamined Patent Application Publication No. 05-213762
Patent Document 5: Japanese unexamined Patent Application Publication No. 02-87979
Patent Document 6: Japanese unexamined Patent Application Publication No. 04-210639
Patent Document 7: Japanese unexamined Patent Application Publication No. 06-70720
Patent Document 8: Japanese Patent No. 4786866

### Non-patent Documents

Non-patent Document 1: Bri. J. Nutr. 75, 461-469, (1996)
Non-patent Document 2: J. Nutr. Biochem. 9, 613-620, (1998)
Non-patent Document 3: Am. J. Physiol. Cell Physiol. 287, C1173-1183, (2004)
Non-patent Document 4: Am. J. Physiol. Gastrointest. Liver Physiol. 292, G647-656, (2007)

### Summary of the Invention

### Object to be Solved by the Invention

An object of the present invention is to provide an agent for use in preventing or ameliorating renal failure.

### Means to Solve the Object

The present inventor has continued intensive studies for solving the above-problems. In these processes, the present inventor has studied the prevention or amelioration effect of lubiprostone (trade name: Amitiza) known as a constipation- ameliorating agent on renal dysfunction; as a result, it has been found that lubiprostone is excellent in the inhibitory action on the advance of renal dysfunction, the suppressive action on renal fibrosis, the suppressive action on the development of renal failure, and the suppressive action on nephritis.

Thus, the present invention relates to (1) lubiprostone or a pharmacologically acceptable salt thereof (the agent) for use in preventing or ameliorating renal failure and (2) the agent for use according to "(1)", wherein the agent is orally administered.

Other embodiments of the present disclosure can include a method for preventing or ameliorating (treating) renal dysfunction by administering the agent for preventing or ameliorating renal dysfunction to a patient in need of the prevention or amelioration (treatment) of the renal dysfunction, and use of lubiprostone or a pharmacologically acceptable salt thereof for use in the prevention or amelioration (treatment) of renal dysfunction and lubiprostone or a pharmacologically acceptable salt thereof for preparing the agent for preventing or ameliorating renal dysfunction.

### Effect of the Invention

The use of lubiprostone or a pharmacologically acceptable salt thereof can inhibit the progression of renal dysfunction, suppress renal fibrosis, suppress the development of renal failure, and suppress nephritis, and thus can prevent or ameliorate nephritides, such as acute glomerulonephritis, minimal change nephritis, chronic glomerulonephritis, renal sclerosis, membrane proliferative nephritis, mesangial proliferative glomerulonephritis, membrane proliferative glomerulonephritis, crescentic nephritis, rapidly progressive glomerulonephritis, membranous glomerulonephritis, tubulointerstitial nephritis, acute pyelonephritis, chronic pyelonephritis, endocapillary proliferative nephritis, and lupus nephritis; renal failures, such as acute renal failure and chronic renal failure; and renal dysfunctions, such as amyloid kidney, membranous nephropathy, focal glomerulosclerosis, IgA nephropathy, acute tubular necrosis, nephrotic syndrome, diabetic nephropathy, gouty kidney, renal edema, renal tumors, renal ischemic disorders, and renal ischemia-reperfusion injury.

### Brief Description of Drawings

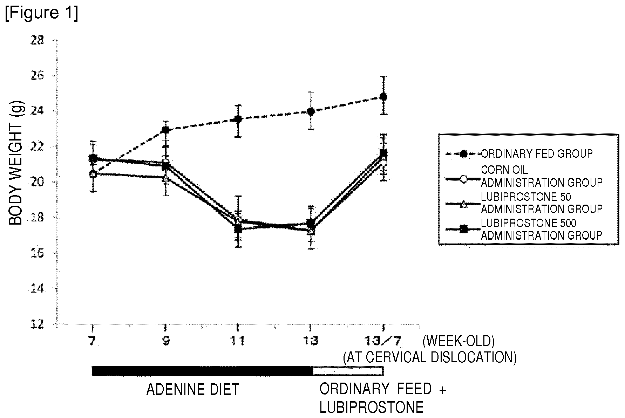

[Figure 1] Figure 1 is a graph showing the results of measuring the body weight change in chronic renal failure model mice to which lubiprostone was administered.

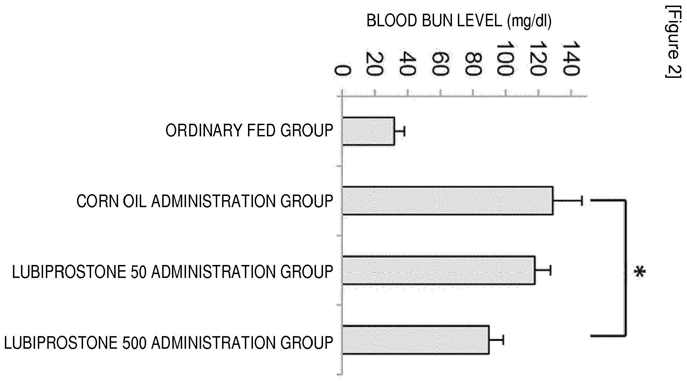

[Figure 2] Figure 2 is a graph showing the results of measuring the level of blood urea nitrogen (BUN) in chronic renal failure model mice to which lubiprostone was administered. The ordinate represents the level (mg/dl) of BUN in the blood (mean ± standard deviation, [n = 6 to 7]). In the figure, "*" shows that a statistically significant difference (p < 0.05) exists by Dunnett test between the corn oil administration group and the lubiprostone 500 administration group.

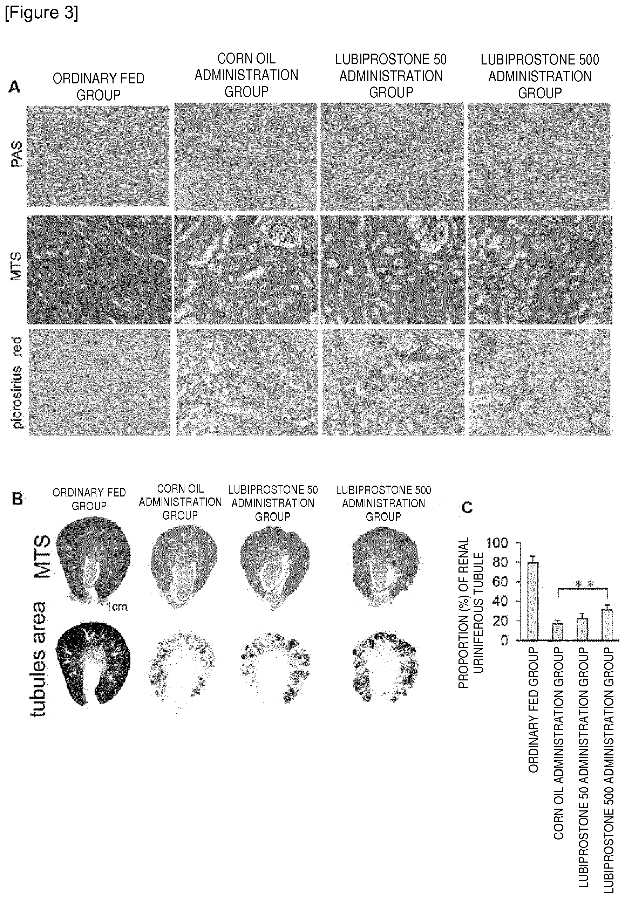

[Figure 3] Figure 3 is a series of drawings showing the results of analyzing the renal tissue of chronic renal failure model mice to which lubiprostone was administered. Panel A in Figure 3 shows the results of staining the renal tissue; panel B shows the results of staining the whole kidney; and panel C shows the results of quantifying the level of staining of the whole kidney. The ordinate in panel C represents the proportion (%) of the renal uriniferous tubule in the whole kidney (mean ± standard deviation, [n = 6 to 7]). In panel C, "**" shows that a statistically significant difference (p < 0.01) exists by Dunnett test between the corn oil administration group and the lubiprostone 500 administration group.

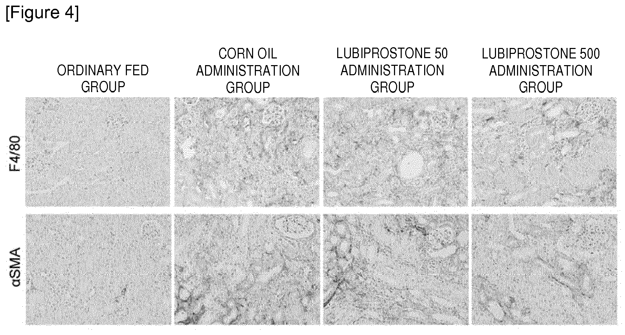

[Figure 4] Figure 4 is a series of photographs showing the results of analyzing the expression of two renal failure marker (F4/80 and αSMA) proteins by DAB staining method in the renal tissue of chronic renal failure model mice to which lubiprostone was administered.

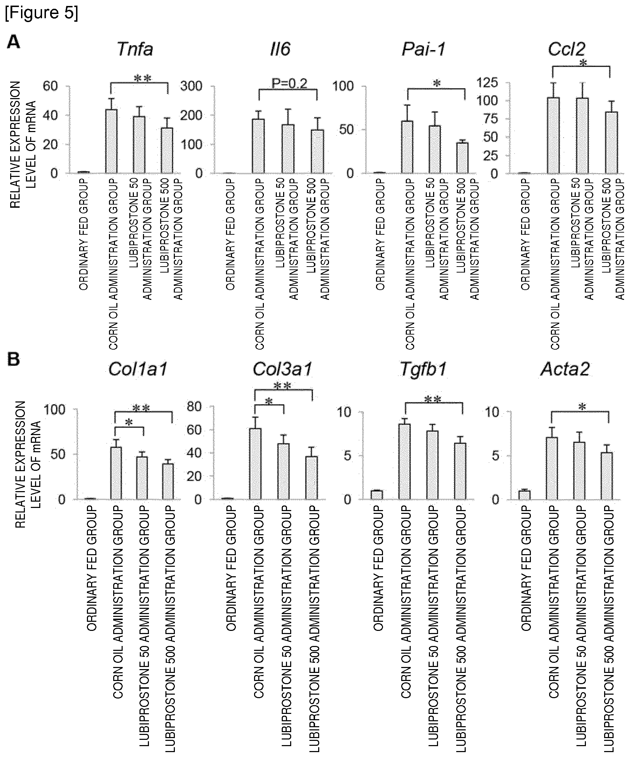

[Figure 5] Figure 5 is a series of graphs showing the results of analyzing the mRNA expression of genes of 8 renal failure markers (4 nephritis-related markers [Tnfa, I16, Pai-1, and Ccl2] [panel A] and 4 fibrosis-related markers [Col1a1, Col3a1, Tgfb1, and Acta2] [panel B]) by a quantitative PCR method in the renal tissue of chronic renal failure model mice to which lubiprostone was administered. The ordinates each represent the relative expression level of mRNA (mean ± standard deviation, [n = 6 to 7]). In the figure, "*" shows that a statistically significant difference (p < 0.05) exists by Dunnett test between the corn oil administration group and the lubiprostone 50 administration group or the lubiprostone 500 administration group, and "**" shows that a statistically significant difference (p < 0.01) exists by Dunnett test between the corn oil administration group and the lubiprostone 500 administration group.

### Mode of Carrying Out the Invention

The agent for preventing or ameliorating renal failure according to the present invention comprises lubiprostone or its pharmacologically acceptable salt (hereinafter collectively referred to as "lubiprostones") as an active ingredient, and, if necessary, compounding ingredients can further be added, such as a pharmaceutically acceptable common carrier, a binder, a stabilizer, an excipient, a diluent, a pH buffering agent, a disintegrator, an isotonic agent, an additive, a coating, a solubilizer, a lubricating agent, a sliding agent, a solubilizing agent, a lubricant, a seasoning, a sweetening agent, a solvent, a gelatinizer, and a nutrient. Specific examples of such compounding ingredients can include water, physiological saline, animal fat and oil, vegetable oil, lactose, starch, gelatin, crystalline cellulose, gum, talc, magnesium stearate, hydroxypropylcellulose, polyalkylene glycol, polyvinyl alcohol, and glycerin.

Lubiprostone is (-)-7-[(2R,4aR,5R,7aR)-2-(1,1-difluoropentyl)-2-hydroxy-6-oxooctahydrocyclopenta[b]-pyran-5-yl]heptanoic acid, and is typically present in the form of the following tautomer.

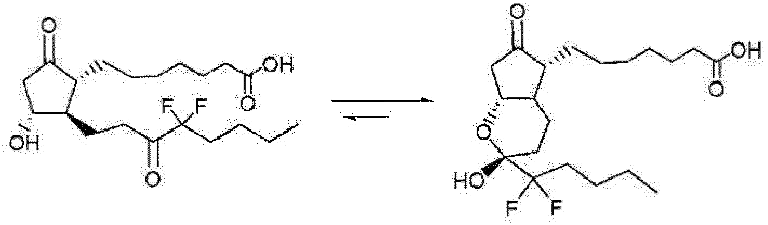

Examples of the pharmacologically acceptable salt of lubiprostone include a metal salt formed from aluminum, calcium, lithium, magnesium, potassium, sodium, or zinc, and an organic salt formed from N,N'-dibenzylethylenediamine, chloroprocaine, choline, diethanolamine, ethylenediamine, N-methylglucamine, lysine, or procaine.

The agent for preventing or ameliorating renal failure according to the present invention can be provided as a dosage form for oral administration for which administration is performed using a formulation, such as a powder, granule, tablet, capsule, syrup, or suspension formulation, and for parenteral administration for which a formulation, such as a solution, emulsion, or suspension formulation, is injected or for intranasal administration using the formulation of a spray; preferred is oral administration.

The dose of the agent for preventing or ameliorating renal dysfunction is properly determined depending on age, body weight, sex, symptom, drug sensitivity, and the like. The agent is typically administered in the dose range of 1 µg to 200 mg/day, preferably in the dose range of 2 µg to 2,000 µg/day, more preferably 3 to 200 µg/day, still more preferably in the dose range of 4 to 20 µg/day, once daily or a plurality of times (e.g., 2 to 4 times) daily in divided doses; however, the dose can be regulated depending on the situation of amelioration of symptoms. The lubiprostone as the active ingredient is an agent (trade name: Amitiza) already used in chronic idiopathic constipation patients and its dosage regimen and side effects are thoroughly known; thus, the dose and the dosage form can also be selected based on such experiences in using the agent for preventing or ameliorating renal failure.

The renal dysfunction indicated for the agent for preventing or ameliorating renal dysfunction according to the present disclosure is not particularly limited provided that it is a condition in which some anomaly occurs in the kidney due to disease, injury, or the like to cause trouble in renal function; examples thereof can include nephritis, such as acute glomerulonephritis, minimal change nephritis, chronic glomerulonephritis, renal sclerosis, membrane proliferative nephritis, mesangial proliferative glomerulonephritis, membrane proliferative glomerulonephritis, crescentic nephritis, rapidly progressive glomerulonephritis, membranous glomerulonephritis, tubulointerstitial nephritis, acute pyelonephritis, chronic pyelonephritis, endocapillary proliferative nephritis, or lupus nephritis; renal failure, such as acute renal failure or chronic renal failure; and amyloid kidney, membranous nephropathy, focal glomerulosclerosis, IgA nephropathy, acute tubular necrosis, nephrotic syndrome, diabetic nephropathy, gouty kidney, renal edema, renal tumors, renal ischemic disorders, and renal ischemia-reperfusion injury. Among these, nephritis and renal failure are preferable because the above-described lubiprostones are each excellent in the suppressive action on the development of nephritis and renal failure.

The lubiprostones are each excellent in the suppressive action on renal fibrosis and the inhibitory action on the advance of renal dysfunction. Thus, the agent for preventing or ameliorating renal dysfunction, which comprises the lubiprostone as an active ingredient, can be advantageously applied to an agent for preventing or ameliorating renal fibrosis and an agent for inhibiting (suppressing) the advance of renal dysfunction.

The effect of each of the lubiprostones reduces the expression level of various inflammatory markers in cells; thus, the agent for preventing or ameliorating renal dysfunction turns out to be also useful for the prevention or treatment of an inflammatory disease accompanying renal dysfunction. Thus, the renal dysfunction includes, for convenience, an inflammatory disease accompanying renal dysfunction. Examples of the inflammatory disease can include rheumatism, inflammatory bowel disease, and hepatitis.

The lubiprostones can each be produced by any of known methods, such as chemical synthesis, production using bacteria, and production using enzymes; however, a commercial product can also be used. Examples of the commercial product can include Amitiza capsule (from Abbot Japan Co., Ltd.) and SPI-0211 (from Sucampo Pharmaceuticals Inc.).

The present invention will be more specifically described below with reference to Examples. However, these Examples are not intended to limit the technical scope of the present invention. In Examples, to confirm that the administration of lubiprostone (trade name: Amitiza) could ameliorate renal function, chronic renal failure model mice were prepared by causing mice to eat adenine and analysis was performed using the chronic renal failure model mice. The chronic renal failure model mice are mice in which nephropathy occurs by the crystallization of the eaten adenine in the uriniferous tubule in the form of insoluble 2,8-dihydroxyadenine (Cozzolino, M. et al. Kidney Int. 64, 441-450 (2003) and Tamagaki, K. et al. Nephrol. Dial. Transplant 21, 651-659 (2006)). All experimental animals were operated after approval by the Ethics Committee of Tohoku University School of Medicine.

### Example 1

### 1. Preparation of Chronic Renal Failure Model Mouse to Which Lubiprostone Was Administered

Chronic renal failure model mice to which lubiprostone was administered were prepared according to a method consisting of the following processes [1] to [4] . The body weight of mice was measured on a weekly basis, and blood pressure was measured by a tailcuff method.
[1] Male C57BL/6 mice (purchased from Clea Japan, Inc.) were fed on an ordinary feed (CE-2 from Clea Japan, Inc.) until 6 weeks of age.
[2] At 7 weeks of age, the mice were divided into an ordinary fed group fed on the ordinary feed (control)and an adenine-diet fed group fed on an ordinary feed containing 0.2% adenine (from Wako) (adenine-diet).
[3] The adenine-diet fed group was fed by returning the feed to the ordinary feed after 6 weeks of adenine-diet feeding (at 13 weeks of age) and divided into 3 administration groups ("corn-oil administration group" [vehicle], "lubiprostone 50 administration group" [Lubi50], and "lubiprostone 500 administration group" [Lubi500]; corn oil, 50 µg/kg (mouse body weight) of lubiprostone dissolved in corn oil [Amitiza capsule] [from Abbot Japan Co., Ltd.], and 500 µg/kg (mouse body weight) of lubiprostone dissolved in corn oil [from Abbot Japan Co., Ltd.] were forcibly orally administered using a sound to the "corn-oil administration group", "lubiprostone 50 administration group", and "lubiprostone 500 administration group", respectively once daily for 12 days.
[4] After 24 hours, the mice were subjected to cervical dislocation.

### Example 2

### 2. Measurement of Body Weight of Chronic Renal Failure Model Mouse to Which Lubiprostone Was Administered

In the 4 groups (the ordinary fed group, the corn-oil administration group, the lubiprostone 50 administration group, and the lubiprostone 500 administration group), the body weight of mice at 7 weeks of age (immediately after adenine-diet feeding), at 9 weeks of age (at 2 weeks of age after adenine-diet feeding), at 11 weeks of age (at 4 weeks of age after adenine-diet feeding), at 13 weeks of age (immediately after lubiprostone administration), and at cervical dislocation (at 13/7 [≈ 1.86] weeks after lubiprostone administration) was measured; the results are shown in Figure 1. As a result, the body weight of the chronic renal failure model mice decreased due to adenine-diet feeding was recovered by returning the feed to the ordinary feed (see "corn oil administration group" in Figure 1); in this regard, it was confirmed that lubiprostone administration did not affect the recovery of body weight (see "lubiprostone 50 administration group" and "lubiprostone 500 administration group" in Figure 1).

### Example 3

### 3. Measurement of BUN Level in Blood of Chronic Renal Failure Model Mouse to Which Lubiprostone Was Administered

Blood was collected according to an established method from the chronic renal failure model mice to which lubiprostone was administered prepared by the method described in Example 1, and the level of BUN in the blood of the mice was measured using i-STAT (from Fuso) (Figure 2). As a result, an increase in the blood BUN level as an index for renal dysfunction was observed in the chronic renal failure model mice (comparison between "corn oil administration group" and "ordinary fed group" in Figure 2) whereas the increase in the blood BUN level was suppressed in a manner dependent on the dose of lubiprostone in the chronic renal failure model mice to which lubiprostone was administered (comparison of "corn oil administration group" with "lubiprostone 50 administration group" or "lubiprostone 500 administration group" in Figure 2). Particularly, a significant difference was noted between the lubiprostone 500 administration group and the corn oil administration group as a control. This result shows the administration of lubiprostone can prevent or ameliorate renal dysfunction and also suggests that it can treat/prevent hypertension caused by renal dysfunction. It was confirmed that the administration of lubiprostone did not change the level of hemoglobin (Hb) in the blood, showing that the suppression of the blood BUN level by lubiprostone administration was not attributable to the dilution of the blood.

### Example 4

### 4. Analysis of Renal Tissue of Chronic Renal Failure Model Mouse to Which Lubiprostone Was Administered

Renal tissue sections were prepared to perform detailed renal tissue analysis. The renal tissue of the chronic renal failure model mice to which lubiprostone was administered prepared by the method described in Example 1 was fixed in 10% neutral-buffer formalin and embedded in paraffin. The paraffin-embedded renal tissue was sliced and analyzed using 3 histological staining methods (periodic acid-Schiff stain [PAS], Masson's trichrome stain [MTS], and picrosirius red stain) (Figure 3). As a result, from the analysis using PAS stain, increased vacuoles and the atrophy or disappearance of the uriniferous tubule were noted in the chronic renal failure model mice (comparison between "corn oil administration group" and "ordinary fed group" in the top [PAS] of panel A in Figure 3) whereas the recovery of the uriniferous tubule was noted in chronic renal failure model mice to which lubiprostone was administered (comparison of "corn oil administration group" with "lubiprostone 50 administration group" or "lubiprostone 500 administration group" in the top [PAS] of panel A in Figure 3).

The staining of collagen fiber using the MTS method resulted in the observation of the fibrosis of the kidney in the chronic renal failure model mice (comparison between "corn oil administration group" and "ordinary fed group" in the middle [MTS] of panel A in Figure 3) whereas the fibrosis of the kidney was suppressed in the chronic renal failure model mice to which lubiprostone was administered (comparison of "corn oil administration group" with "lubiprostone 50 administration group" or "lubiprostone 500 administration group" in the middle [MTS] of panel A in Figure 3).

The staining of collagen fiber using the picrosirius red stain resulted in the observation of the fibrosis of the kidney in the chronic renal failure model mice, similarly to the results obtained using the MTS method (comparison between "corn oil administration group" and "ordinary fed group" in the bottom [Picrosirius Red] of panel A in Figure 3) whereas the fibrosis of the kidney was suppressed in the chronic renal failure model mice to which lubiprostone was administered (comparison of "corn oil administration group" with "lubiprostone 50 administration group" or "lubiprostone 500 administration group" in the bottom [Picrosirius Red] of panel A in Figure 3).

When the whole kidney was subjected to collagen fiber staining using the MTS method, the fibrosis of the kidney was observed in the chronic renal failure model mice, similarly to the results obtained using the renal tissue sections (comparison between "corn oil administration group" and "ordinary fed group" in the top [MTS] of panel B in Figure 3) whereas the fibrosis of the kidney was suppressed in the chronic renal failure model mice to which lubiprostone was administered (comparison of "corn oil administration group" with "lubiprostone 50 administration group" or "lubiprostone 500 administration group" in the top [MTS] of panel B in Figure 3). In addition, the staining of the renal uriniferous tubule using the MTS method resulted in the observation of the marked decrease of the renal uriniferous tubule in the chronic renal failure model mice (comparison between "corn oil administration group" and "ordinary fed group" in the bottom [tubules area] of panel B in Figure 3) whereas the decrease of the renal uriniferous tubule was suppressed in a manner dependent on the dose of lubiprostone in the chronic renal failure model mice to which lubiprostone was administered (comparison of "corn oil administration group" with "lubiprostone 50 administration group" or "lubiprostone 500 administration group" in the bottom [tubules area] of panel B in Figure 3, and panel C in Figure 3). Particularly, a significant difference was noted between the lubiprostone 500 administration group and the corn oil administration group as a control (panel C in Figure 3).

The above results supported the results of the above Example 3, and the analysis of the kidney at the tissue level confirmed that the administration of lubiprostone could suppress the fibrosis of the kidney and the decrease of the renal uriniferous tubule.

### Example 5

### 5. Expression Analysis of Renal Failure Marker in Renal Tissue of Chronic Renal Failure Model Mouse to Which Lubiprostone Was Administered

F4/80 and αSMA are known as renal failure markers. Accordingly, using the expression of these renal failure marker proteins as an index, it was confirmed that the administration of lubiprostone suppressed the advance of renal failure. The DAB staining method was performed according to an established method using the renal tissue sections prepared in Example 4 and an anti-F4/80 antibody (from Serotec Co., Ltd.) and an anti-aSMA antibody (from DAKO Co., Ltd.) (Figure 4). As a result, an increase in the expression of the 2 renal failure markers (F4/80 and αSMA) was observed in the chronic renal failure model mice (comparison between "corn oil administration group" and "ordinary fed group" in the upper [F4/80] in Figure 4 and comparison between "corn oil administration group" and "ordinary fed group" in the lower [αSMA]) whereas the increase in the expression of the 2 renal failure markers was suppressed in the chronic renal failure model mice to which lubiprostone was administered (comparison between "corn oil administration group" with "lubiprostone 50 administration group" or "lubiprostone 500 administration group" in the upper[F4/80] in Figure 4 and comparison between "corn oil administration group" with "lubiprostone 50 administration group" or "lubiprostone 500 administration group" in the lower [αSMA]).

In addition, 8 renal failure markers other than F4/80 and αSMA (4 nephritis-related markers [Tnfa, Il6, Pai-1, and Ccl2] and 4 fibrosis-related markers [Col1a1, Col3a1, Tgfb1, and Acta2]) were also analyzed. The whole kidney of each of the chronic renal failure model mice to which lubiprostone was administered, prepared in Example 1 was homogenized; the total RNA was extracted/purified using Trizol reagent (from Invitrogen Co., Ltd.); and cDNA was prepared using an oligo(dT) primer and Transcriptor first strand cDNA synthesis kit (from Roche Co., Ltd.). The expression level of mRNA of genes of the 8 renal failure markers (the 4 nephritis-related markers and the 4 fibrosis-related markers) was detected by performing quantitative PCR using Fast start universal probe master (from Roche Co., Ltd.) under the conditions described in the following 1) to 2). GAPDH gene was used as an internal standard.

1) 1 cycle at 95°C for 10 minutes (activation of polymerase)
2) 40 cycles of reciprocation between 95°C for 15 seconds and 60°C for 1 minute (amplification of cDNA using "forward primer" and "reverse primer")

TaqMan Gene Expression Assay probe/primer sets (from Applied Biosystems Co., Ltd.) were used as primer/probe sets for amplifying and detecting cDNA of the genes of the 8 renal failure markers (the 4 nephritis-related markers and the 4 fibrosis-related markers) (see Table 1).

**[Table 1]**

| Gene Name | Assay ID |
|---|---|
| Nephritis-related Marker Gene | |
| Tnfa | Mm00443260_gl |
| Il6 | Mm00446190_ml |
| Pai-1 | Mm00435860_ml |
| Ccl2 | Mm00441242_ml |

| Fibrosis-related Marker Gene | |
|---|---|
| Col1a1 | Mm00801666_gl |
| Col3a1 | Mm01254476_ml |
| Tgfb1 | Mm01178820_ml |
| Acta2 | Mm00725412_sl |

| Internal Standard Gene | |
|---|---|
| GAPDH | Mm99999915_gl |

The number of PCR cycles at which each PCR product reaches a certain amount (threshold cycle: Ct value) was measured using Baseline software (from Applied Biosystems Co., Ltd.); the relative Ct value of the cDNA amplification product of the gene of each of the 8 renal failure markers (the 4 nephritis-related markers and the 4 fibrosis-related markers) based on the Ct value of the cDNA amplification product of the GAPDH gene was determined by a comparative Ct method (delta-delta Ct method); and the relative amount of cDNA of the gene of each of the 8 renal failure markers (the 4 nephritis-related markers and the 4 fibrosis-related markers), i.e., the relative amount of mRNA of the gene of each of the 8 renal failure markers (the 4 nephritis-related markers and the 4 fibrosis-related markers), was calculated from the relative Ct value (see the ordinate of Figure 5). As a result, it was shown that whereas the expression level of mRNA of all of the genes of the 8 renal failure markers (the 4 nephritis-related markers and the 4 fibrosis-related markers) increased in the chronic renal failure model mice (comparison between "corn oil administration group" and "ordinary fed group", in each graph of Figure 5), the administration of lubiprostone decreased the expression level of mRNA of all of the genes of the 8 renal failure markers (the 4 nephritis-related markers and the 4 fibrosis-related markers) in a manner dependent on the dose of lubiprostone in the chronic renal failure model mice (comparison of "corn oil administration group" with "lubiprostone 50 administration group" or "lubiprostone 500 administration group" in Figure 5). Particularly, a significant difference was noted between the lubiprostone 500 administration group and the corn oil administration group as a control for the genes of the 7 renal failure markers (the 3 nephritis-related markers and the 4 fibrosis-related markers), excluding Il6 (Figure 5).

The above results supported the results of the above Examples 3 and 4, and the analysis of the renal failure markers at the expression level confirmed that the administration of lubiprostone could prevent the development of nephritis and renal failure. Since the administration of lubiprostone decreased the expression level of various inflammatory markers, it was suggested that lubiprostone was also effective in preventing or treating various inflammatory diseases (e.g., rheumatism, inflammatory bowel disease, and hepatitis) accompanying renal dysfunction.

### Industrial Applicability

Lubiprostone or a pharmacologically acceptable salt thereof can inhibit the progression of renal dysfunction, suppress renal fibrosis, suppress the development of renal failure, and suppress nephritis, and thus is conductive to the development of a therapeutic agent for preventing an increase in the severity of renal dysfunction or a therapeutic agent for slowing the aggravation of renal dysfunction to the stage requiring the introduction of dialysis.

## Claims

1. Lubiprostone or a pharmacologically acceptable salt thereof for use in the prevention or amelioration of renal failure.

2. The lubiprostone or a pharmacologically acceptable salt thereof for use according to claim 1, which is orally administered.

## Patentansprüche

1. Lubiproston oder ein pharmakologisch annehmbares Salz davon zur Verwendung bei der Vorbeugung oder Linderung von Nierenversagen.

2. Lubiproston oder ein pharmakologisch annehmbares Salz davon zur Verwendung nach Anspruch 1, das oral verabreicht wird.

## Revendications

1. Lubiprostone ou un sel pharmacologiquement acceptable de celui-ci pour une utilisation dans la prévention ou l'amélioration d'une insuffisance rénale.

2. Lubiprostone ou un sel pharmacologiquement acceptable de celui-ci pour une utilisation selon la revendication 1, qui est administré oralement.
